(19) Europäisches Patentamt
European Patent Office
Office européen des brevets

(11) Veröffentlichungsnummer: **0 075 680**
**B1**

(12) # EUROPÄISCHE PATENTSCHRIFT

(45) Veröffentlichungstag der Patentschrift: **20.11.86**

(51) Int. Cl.⁴: **C 07 D 309/32**, C 07 D 307/32

(21) Anmeldenummer: **82107030.7**

(22) Anmeldetag: **04.08.82**

(54) **Substituierte Furanone sowie Verfahren zur Herstellung von 3,4-Dihydro-alpha-pyronen und substituierten Furanonen.**

(30) Priorität: **30.09.81 DE 3138843**

(43) Veröffentlichungstag der Anmeldung:
**06.04.83 Patentblatt 83/14**

(45) Bekanntmachung des Hinweises auf die Patenterteilung:
**20.11.86 Patentblatt 86/47**

(84) Benannte Vertragsstaaten:
**AT BE CH DE FR GB IT LI LU NL SE**

(56) Entgegenhaltungen:
**EP - A - 0 031 932**
**EP - A - 0 036 131**
**US - A - 2 428 015**
**US - A - 4 031 115**

(73) Patentinhaber: **DYNAMIT NOBEL AKTIENGESELLSCHAFT, Postfach 1209, D-5210 Troisdorf, Bez. Köln (DE)**

(72) Erfinder: **Schmidt, Hans-Georg, Dr., Porzer Strasse 15, D-5216 Niederkassel-Ranzel (DE)**

EP 0 075 680 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein neues Herstellungsverfahren zur Gewinnung von 3,4-Dihydro-α-pyronen der Formel

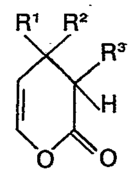

I,

worin der $R^1$, $R^2$ und $R^3$ gleich oder verschieden sein können und für Wasserstoff oder Alkylgruppen mit 1 bis 10 C-Atomen stehen, sowie die als Ausgangsstoffe dienenden neuen Verbindungen der Formel

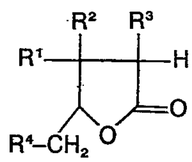

IIa, b

worin $R^1$, $R^2$ und $R^3$ die vorstehende Bedeutung haben und $R^4$ die Reste

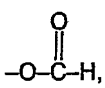 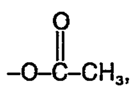 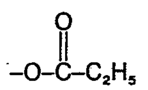 oder $-O-R^5$

bedeutet, wobei $R^5$ für niedere Alkylgruppen steht.

Weiter betrifft die Erfindung Stoffe der Formel II und Verfahren zu deren Herstellung.

Verbindungen der Formel I sind aus der DE-OS 2 952 068 bekannt, jedoch erfolgt dort die Herstellung aus substituierten Dihydro-5-hydroxymethyl-furanonen, für die ein aufwendiges Herstellungsverfahren genannt wird.

Gegenstand der Erfindung ist ein Verfahren zur Herstellung von 3,4-Dihydro-α-pyronen der Formel

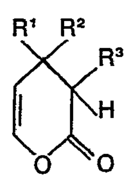

I,

worin $R^1$, $R^2$ und $R^3$ gleich oder verschieden sein können und für Wasserstoff oder Alkylgruppen mit 1 bis 10 C-Atomen stehen, welches dadurch gekennzeichnet ist, dass man eine Verbindung der Formel

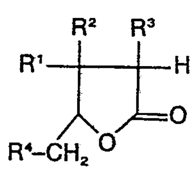

II,

worin $R^1$, $R^2$ und $R^3$ die vorstehende Bedeutung haben und $R^4$ die Reste

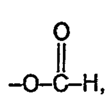 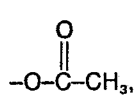 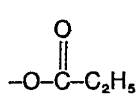

(Formel IIa) oder $-O-R^5$ (Formel IIb) bedeutet, wobei $R^5$ für niedere Alkylgruppen steht, erhitzt.

Bevorzugt ist verfahrensgemäss der Rest

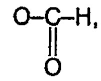

in zweiter Linie der Rest

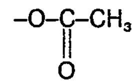

Die Niederalkylgruppen $R^5$ sollen 1 bis 4, bevorzugt 1 bis 3 C-Atome enthalten.

Bevorzugt sind von den Resten $R^1$, $R^2$ und $R^3$ ein oder zwei Reste Alkylreste.

Vorteil des Verfahrens ist die leichte Zugänglichkeit der neuen Stoffe der Formel II bzw. die Vermeidung einer Stufe bei der Herstellung sowie hohe Ausbeuten und gute Reinheit.

Die Umsetzung ist im Temperaturbereich von 150 bis etwa 650 °C möglich, wird jedoch bevorzugt im Temperaturbereich von 200 bis 500 °C durchgeführt. Wählt man die Temperatur niedriger, so ist der Umsatz langsam.

Zweckmässigerweise tropft man die Ausgangsverbindung durch ein erhitztes, mit temperaturbeständigem Material gefülltes Rohr. Die Umsetzung wird bevorzugt in einer Inertgasatmosphäre bei Normaldruck oder vermindertem Druck, allgemein zwischen $10^{-3}$ und 1 bar durchgeführt. Das temperaturbeständige Füllmaterial kann ein inerter Stoff, wie Glas, Keramik oder dergleichen sein. Bevorzugt werden als Füllmaterial Silikate und Aluminiumsilikate, wie Montmorillonite oder Zeolithe; diese wirken als Katalysatoren, so dass bereits bei Temperaturen von 200 bis 300 °C gute Umsätze erreicht werden.

Die Ausgangsstoffe können in Mischung miteinander oder auch in Mischung mit Dihydro-5-hydroxymethyl-furanonen der Formel

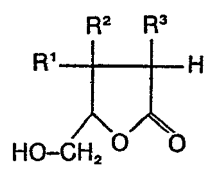

IIc

eingesetzt werden, wobei die Substituenten $R^1$ bis $R^3$ mit denen der Formel II identisch sind.

Die Ausgangsverbindungen können in Substanz oder in Lösung eingesetzt werden. Als Lösungsmittel kommen temperaturstabile Verbindungen wie z. B. Benzol, Toluol, Xylol bevorzugt in Frage. Die Aufarbeitung und Reinigung der Verbindungen der Formel I erfolgt bevorzugt durch Destillation.

Weiterer Gegenstand sind die neuen Verbindungen der Formel

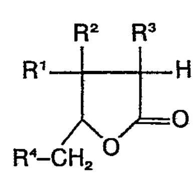

II,

worin $R^1$, $R^2$ und $R^3$ gleich oder verschieden sein

können und für Wasserstoff oder Alkylgruppen mit 1 bis 10 C-Atomen stehen, wobei im Falle der Ester der Formel IIa mindestens einer der Reste $R^1$, $R^2$ oder $R^3$ eine solche Alkylgruppe ist und $R^4$ die Reste

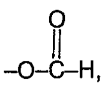, 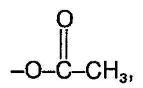, 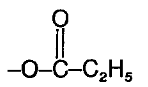 oder $-O-R^5$

bedeutet, wobei $R^5$ für niedere Alkylgruppen steht.

Der Rest $R^5$ hat bevorzugt 1 bis 4 Kohlenstoffatome.

Die Ester der Ameisen-, Essig- und Propionsäure der Formel IIa können aus Penten-4-säureestern der Formel

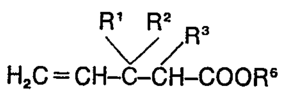 III,

worin $R^1$, $R^2$ und $R^3$ dieselbe Bedeutung wie in Formel IIa haben und $R^6$ Alkylreste mit 1 bis 10, vorzugsweise 1 bis 4 C-Atomen oder Alkenylreste mit 2 bis 10 C-Atomen sind, durch Umsetzung mit Wasserstoffperoxid und Ameisen-, Essig- bzw. Propionsäure hergestellt werden. Die bevorzugte Säure ist die Ameisensäure.

Die Carbonsäuren können in Mengen von 2 bis 20 Mol je Mol Pentensäureester verwendet werden. Wasserstoffperoxid kann als wässrige Lösung eingesetzt werden. Es soll 0,9 bis 1,1 Mol $H_2O_2$ je Mol Pentensäureester verwendet werden. Die Temperaturen liegen zwischen 20 °C und dem Siedepunkt der Reaktionsmischung.

Die Verbindungen der Formel IIb mit $R^4 = OR^5$ erhält man durch Umsetzung von Dihydro-5-halogenmethyl-furanonen der Formel

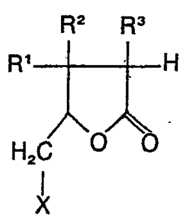 IV, X = Cl, Br

in der $R^1$, $R^2$ und $R^3$ Alkylreste mit 1 bis 10 C-Atomen oder H bedeuten, mit Basen und Alkoholen der Formel $R^5OH$.

Geeignete Basen sind die Alkali- bzw. Erdalkali-Hydroxyde, -Oxyde, -Carbonate und Alkali- bzw. Erdalkalialkoholate der Alkohole $R^5OH$ in mindestens äquivalenter Menge. Bevorzugt sind die Alkoholate.

Ein Überschuss von 0,1 bis 1 Äquivalenten Base ist zweckmässig. Die Temperaturen liegen zwischen 20 °C und dem Siedepunkt des Reaktionsgemisches.

Im Rahmen der Erfindung sind unter den Alkylresten Methyl und Äthyl bevorzugt. Weiter sind ein oder zwei der Reste $R^1$, $R^2$ und $R^3$ bevorzugt Wasserstoff.

Die substituierten Penten-4-säureester sind durch Umsetzung von entsprechend substituierten Allylalkoholen mit entsprechend substituierten Orthoessigsäureestern zugänglich (Tetrahedron Letters (1977), Seite 2543).

Die substituierten Dihydro-5-halogenmethylfuranone können entsprechend der DE-OS 2 952 068 erhalten werden.

Die substituierten Pyrone und Furanone sind wichtige Zwischenprodukte für u.a. Insektizide.

Sehr bevorzugt werden die Carbonsäuren gemäss dem Anspruch 7 nur in Mengen von 2,1 bis 2,5 Mol je Mol Pentensäureester eingesetzt, was zusammen mit der ebenfalls geringen Menge $H_2O_2$ eine wesentliche Vereinfachung und Stoffersparnis gegenüber dem Stand der Technik darstellt. In vorteilhafter Weise ist dann auch nur ein geringer Überschuss an Carbonsäure nach der Reaktion zu entfernen.

Sehr bevorzugt ist es weiter möglich, $H_2O_2$ als nur 30 bis 35 Gew.-%ige wässrige Lösung einzusetzen, wodurch keine Gefahr einer Explosion mehr besteht.

Vor der Destillation der Ester der Formel IIa ist es zweckmässig aus Gründen der Sicherheit restliches $H_2O_2$ bzw. gegebenenfalls entstandene Peroxide zu zerstören. Das kann beispielsweise durch Zugabe von Eisen-II-Salzen wie $FeSO_4$ als Lösung in Wasser geschehen. Die Menge $FeSO_4$ ist auf die gemessene Menge an Peroxiden abzustimmen.

Die 3,4-Dihydro-furanone mit Substituenten in 5-Stellung und gegebenenfalls Substituenten in weiteren Positionen können auch als Dihydro-2(3H)-furanone bezeichnet werden.

Beispiel 1:
3,4-Dihydro-4,4-dimethyl-α-pyron

60 g des Ameisensäureesters des
Dihydro-4,4-dimethyl-5-hydroximethyl-(3H)-furanons

werden durch ein senkrecht stehendes Glasrohr (Länge 30 cm, Durchmesser 2,2 cm), welches mit Montmorillonit-Kugeln gefüllt ist und durch äussere Beheizung eine innere Temperatur von 300 °C aufweist, getropft. Die Zutropfzeit beträgt 2 Stunden. Das thermolysierte Material wird in einer gekühlten Vorlage aufgefangen. Nach fraktionierter Destillation erhält man 31 g 3,4-Dihydro-4,4-dimethyl-α-pyron.

Beispiel 2:
3,4-Dihydro-4,4-dimethyl-α-pyron

20 g des Essigsäureesters des
Dihydro-4,4-dimethyl-5-hydroximethyl-(3H)-furanons

werden in der im Beispiel 1 beschriebenen Art und Weise thermolysiert. Nach fraktionierter Destillation und Ausschütteln der Hauptfraktion, die bei 65 bis 70 °C (12 mm) 0,09 übergeht, mit Wasser um restliche Essigsäure zu entfernen, enthält man 9,7 g 3,4-Dihydro-4,4-dimethyl-α-pyron.

Beispiel 3:
3,4-Dihydro-4,4-dimethyl-α-pyron

13 g des
Dihydro-4,4-dimethyl-5-methoximethyl-(3H)-furanons

werden wie im Beispiel 1 thermolysiert. Nach fraktionierter Destillation des Roh-Thermolysates erhält man 6,6 g 3,4-Dihydro-4,4-dimethyl-α-pyron.

Beispiel 4:
3,4-Dihydro-α-pyron

Analog zu Beispiel 3 wurden 10 g des Methyläthers des Dihydro-5-hydroximethyl-(3H)-furanons thermolysiert. Das rohe Thermolysat enthält nach gaschromatographischen Untersuchungen und NMR-Analyse 5,1 g 3,4-Dihydro-α-pyron.

Beispiel 5:
Lacton der Formel IIa

109 g 3,3-Dimethylpenten-4-säuremethylester werden zu einer Lösung von 95,5 g $H_2O_2$ (30%ige wässrige Lösung) und 150 g Ameisensäure zugetropft. Dabei steigt die Temperatur des Reaktionsgemisches auf 60 °C. Nach Beendigung der Reaktion (6 Std.) wird das überschüssige $H_2O_2$ mit Eisen-II-sulfat zerstört. Nach Destillation des Rohgemisches erhält man 119,8 g des Ameisensäureesters des
Dihydro-4,4-dimethyl-5-hydroximethyl-(3H)-furanons
(Sdp. 127° C/(0,1 mm) $7{,}5\cdot10^{-4}$ Pa.

NMR-Spektrum (80 MHZ, $C_6D_6$): δ ppm = 0,55 (3H), 0,68 (3H), 1,94 (2H); 3,35–4,13 (4H), 7,65 (1H).

Beispiel 6:
Lacton der Formel IIa

Analog zu Beispiel 5 werden 109 g 3,3-Dimethylpenten-4-säuremethylester miv $H_2O_2$ in 170 g Essigsäure umgesetzt. Nach analoger Aufarbeitung erhält man 128 g des Essigsäureesters des
Dihydro-4,4-dimethyl-5-hydroximethyl-(3H)-furanons.
(Sdp. 126° C/(0,9 mm) $6.75\cdot10^{-3}$ Pa.

NMR-Spektrum (30 MHZ, $CDCl_3$): δ ppm = 1,1 (3H); 1,3 (4H); 2,1 (3H); 2,5 (2H); 4,4 (3H).

Beispiel 7
169 g
Dihydro-4,4-dimethyl-5-brommethyl-(3H)-furanon werden mit 680 ml Methanol und 47 g Natriummethylat versetzt und diese Mischung 7 Std. auf 68 °C erhitzt. Danach wird das Methanol abdestilliert, der Rückstand in $H_2O$ aufgenommen, die wässrige Lösung mit Äther extrahiert. Nach Trocknung der Ätherphase mit Natriumsulfat erhält man nach Entfernen des Lösungsmittels und fraktionierter Destillation des Rückstandes 28 g des
Dihydro-4,4-dimethyl-5-methoximethyl-(3H)-furanons
(Sdp. 156 °C/(33 mm) 0,24 Pa.

NMR-Spektrum (30 MHZ, $CCl_4$): δ ppm = 1,1 (3H); 1,2 (3H); 2,3 (2H); 3,4 (3H); 3,5–4,4 (3H).

**Patentansprüche**

1. Verfahren zur Herstellung von 3,4-Dihydro-α-pyronen der Formel

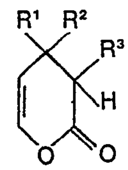

I,

worin $R^1$, $R^2$ und $R^3$ gleich oder verschieden sein können und für Wasserstoff oder Alkylgruppen mit 1 bis 10 C-Atomen stehen, dadurch gekennzeichnet, dass man eine Verbindung der Formel

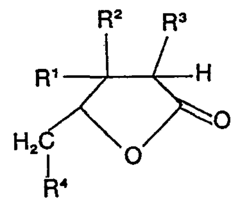

II,

worin $R^1$, $R^2$ und $R^3$ die vorstehende Bedeutung haben und $R^4$ die Reste

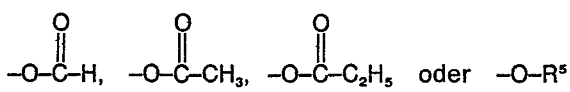

bedeutet, wobei $R^5$ für niedere Alkylgruppen steht, erhitzt.

2. Verfahren nach Anspruch 1, dadurch gekennzeichnet, dass man die Verbindungen der Formel II, auf Temperaturen zwischen 200 und 500 °C erhitzt.

3. Verfahren nach Anspruch 1, dadurch gekennzeichnet, dass die Ausgangsstoffe in Kontakt mit Silikaten oder Aluminosilikate als Katalysatoren eingesetzt werden.

4. Verfahren nach Anspruch 3, dadurch gekennzeichnet, dass als Katalysatoren Zeolithe oder Montmorillonite eingesetzt werden.

5. Verfahren nach mindestens einem der Ansprüche 1 bis 3, dadurch gekennzeichnet, dass die Ausgangsstoffe in Mischung mit entsprechend substituierten Dihydro-5-hydroxymethylfuranonen eingesetzt werden.

6. Substituierte Lactone der Formel

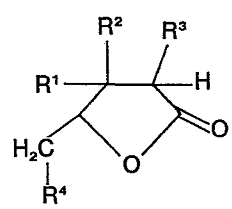

II a,

worin $R^1$, $R^2$ und $R^3$ gleich oder verschieden sein können und für Wasserstoff oder Alkylgruppen mit 1 bis 10 C-Atomen stehen, wobei mindestens einer der Reste $R^1$, $R^2$ oder $R^3$ eine solche Alkylgruppe ist, und $R^4$ die Reste

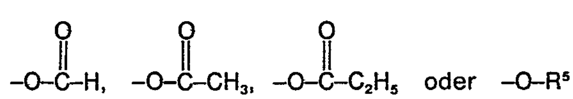

bedeutet.

7. Verfahren zur Herstellung von Lactonen der Formel IIa, worin $R^4$ die Reste

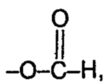, 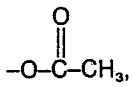, 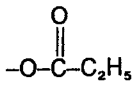

bedeutet, dadurch gekennzeichnet, dass Penten-4-carbonsäureester der Formel

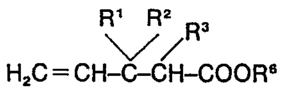 III,

worin $R^1$, $R^2$ und $R^3$, gleich oder verschieden sein können und für Wasserstoff oder Alkylgruppen mit 1 bis 10 C-Atomen stehen, wobei mindestens einer der Reste $R^1$, $R^2$ oder $R^3$ eine solche Alkylgruppe ist, und $R^6$ Alkylreste mit 1 bis 10 C-Atomen oder Alkenylreste mit 2 bis 10 C-Atomen sind, mit Wasserstoffperoxid und Ameisensäure bzw. Essigsäure bzw. Propionsäure umgesetzt werden.

8. Lactone der Formel

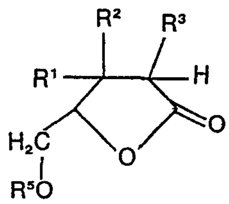 II b,

worin $R^1$, $R^2$ und $R^3$ gleich oder verschieden sind und für Wasserstoff oder Alkylgruppen mit 1 bis 10 C-Atomen stehen und $R^5$ Alkylgruppen mit 1 bis 4 C-Atomen bedeutet.

9. Verfahren zur Herstellung von Lactonen der Formel IIb), worin $R^5$ Alkylgruppen mit 1 bis 4 C-Atomen bedeutet, dadurch gekennzeichnet, dass Dihydro-5-halogenmethylfuranone der Formel

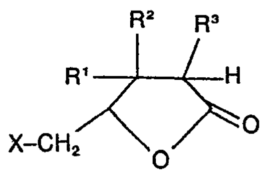 IV,

worin $X = Cl$ oder $Br$ ist und $R^1$, $R^2$ und $R^3$ die im Anspruch 8 genannte Bedeutung haben, mit Basen und Alkoholen der Formel $R^5OH$ umgesetzt werden.

## Claims

1. Process for the production of 3,4-dihydro-α-pyrones of the formula

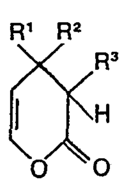 I,

wherein $R^1$, $R^2$ and $R^3$ can be the same or different and stand for hydrogen or alkyl groups with 1 to 10 C-atoms, characterised in that a compound of the formula

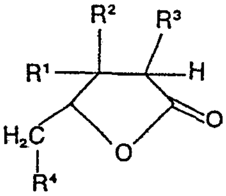 II,

wherein $R^1$, $R^2$ and $R^3$ have the aforementioned meaning and $R^4$ denotes the residues

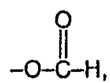, 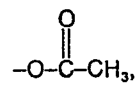, 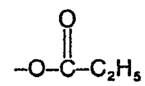 or $-O-R^5$

wherein $R^5$ stands for lower alkyl groups, is heated.

2. Process according to claim 1, characterised in that the compounds of formula II are heated to temperatures between 200 and 500 °C.

3. Process according to claim 1, characterised in that the starting materials are employed in contact with silicates of alumino silicates as catalysts.

4. Process according to claim 3, characterised in that zeolites or montmorillonites are employed as catalysts.

5. Process according to at least one of claims 1 to 3, characterised in that the starting materials are employed in admixture with correspondingly substituted dihydro-5-hydroxymethyl furanones.

6. Substituted lactones of the formula

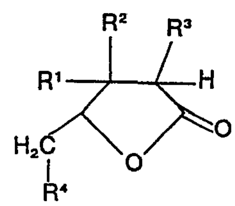 II a,

wherein $R^1$, $R^2$ and $R^3$ can be the same or different and stand for hydrogen or alkyl groups with 1 to 10 C-atoms, wherein at least one of the residues $R^1$, $R^2$ or $R^3$ is such an alkyl group and $R^4$ denotes the residues

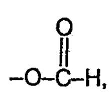, 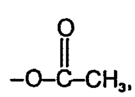, 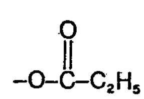 or $-O-R^5$

7. Process for the preparation of lactones of the formula IIa, wherein $R^4$ denotes the residues

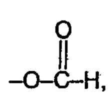, 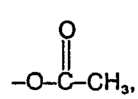, 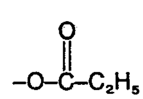 or $-O-R^5$

characterised in that penten-4-carboxylic acid esters of the formula

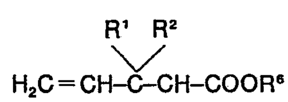 III,

wherein $R^1$, $R^2$ and $R^3$ can be the same or different and stand for hydrogen or alkyl groups with 1 to 10 C-atoms, wherein at least one of the residues $R^1$, $R^2$ or $R^3$ is such an alkyl group and $R^6$ is an

alkyl residue with 1 to 10 C-atoms or alkenyl residue with 2 to 10 C-atoms, is reacted with hydrogen peroxide and formic acid or acetic acid or propionic acid.

8. Lactones of the formula

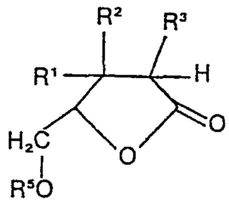

II b,

wherein $R^1$, $R^2$ and $R^3$ are the same or different and stand for hydrogen or alkyl groups with 1 to 10 C-atoms and $R^5$ denotes alkyl groups with 1 to 4 C-atoms.

9. Process for the preparation of lactones of the formula IIb), wherein $R^5$ denotes alkyl groups with 1 to 4 C-atoms, characterised in that dihydro-5-halomethyl furanones of the formula

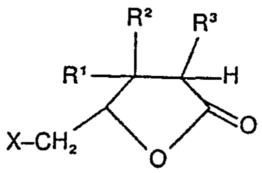

IV,

wherein X = Cl or Br and $R^1$, $R^2$ and $R^3$ have the meaning given in claim 8, are reacted with bases and alcohols of the formula $R^5OH$.

## Revendications

1. Procédé de préparation de 3,4-dihydro-alpha-pyrones de formule:

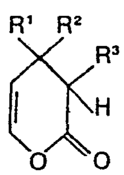

I,

dans laquelle $R^1$, $R^2$ et $R^3$ peuvent être identiques ou différents et représentent un atome d'hydrogène ou des groupes alkyles ayant 1 à 10 atomes de carbone, caractérisé en ce qu'on chauffe un composé de formule:

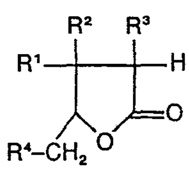

II,

dans laquelle $R^1$, $R^2$ et $R^3$ ont le sens précité et $R^4$ représente les restes

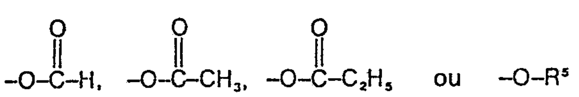

où $R^5$ représente des groupes alkyles inférieurs.

2. Procédé selon la revendication 1, caractérisé en ce qu'on chauffe les composés de formule II jusqu'à des températures comprises entre 200 et 500 °C.

3. Procédé selon la revendication 1, caractérisé en ce qu'on utilise les substances de départ en contact avec des silicates ou des alumino-silicates à titre de catalyseurs.

4. Procédé selon la revendication 3, caractérisé en ce qu'on utilise comme catalyseurs des zéolites ou des montmorillonites.

5. Procédé selon l'une au moins des revendications 1 à 3, caractérisé en ce qu'on utilise les substances de départ en mélange avec des dihydro-5-hydroxyméthylfuranones substituées correspondantes.

6. Lactones substituées de formule:

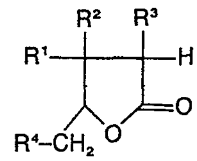

IIa,

dans laquelle $R^1$, $R^2$ et $R^3$ peuvent être identiques ou différents et représentent un atome d'hydrogène ou des groupes alkyles ayant 1 à 10 atomes de carbone, l'un au moins des restes $R^1$, $R^2$ ou $R^3$ représentant un tel groupe alkyle, et $R^4$ désignant les restes

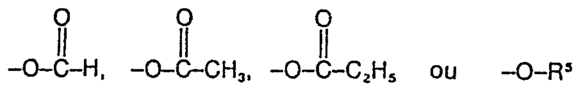

7. Procédé de préparation de lactones de formule IIa, dans laquelle $R^4$ représente les restes

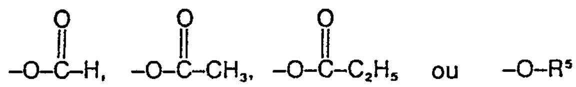

procédé caractérisé en ce qu'on fait réagir des esters d'acides pentène-4 carboxyliques de formule:

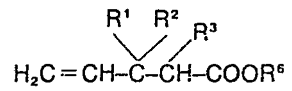

III,

dans laquelle $R^1$, $R^2$ et $R^3$ peuvent être identiques ou différents et représentent un atome d'hydrogène ou des groupes alkyles ayant 1 à 10 atomes de carbone, l'un au moins des restes $R^1$, $R^2$ ou $R^3$ étant un tel groupe alkyle, et $R^6$ représentant des restes alkyles ayant 1 à 10 atomes de carbone ou des restes alcényles ayant 2 à 10 atomes de carbone, avec le peroxyde d'hydrogène et l'acide formique ou l'acide acétique ou l'acide propionique.

8. Lactones de formule:

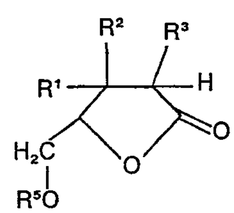

II b,

dans laquelle $R^1$, $R^2$ et $R^3$ sont identiques ou différents et représentent un atome d'hydrogène ou des groupes alkyles ayant 1 à 10 atomes de carbone, et $R^5$ représente des groupes alkyles ayant 1 à 4 atomes de carbone.

9. Procédé de préparation de lactones de formule IIB, dans laquelle $R^5$ représente des groupes alkyles ayant 1 à 4 atomes de carbone, caractérisé en ce qu'on fait réagir des dihydro-5-halogénométhyl-furanones de formule:

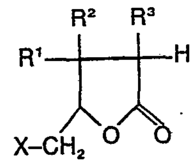

IV,

dans laquelle X représente Cl ou Br et $R^1$, $R^2$ et $R^3$ ont le sens indiqué à la revendication 8, avec des bases et des alcools de formule $R^5OH$.